# EUROPEAN PATENT APPLICATION

(11) **EP 4 737 479 A1**
(43) Date of publication of application: **06.05.2026**
(21) Application number: 24831032.8
(22) Date of filing: 28.06.2024
(51) Int. Cl.: C07K 16/28, C07K 16/30, A61K 39/00

(54) **4-1BB-BINDING PROTEIN**

(30) Priority: 30.06.2023 CN 202310802975
(71) Applicant: Bio-Thera Solutions, Ltd., Guangzhou, Guangdong Province 510005 (CN)
(72) Inventor: LIU, Guosheng, Guangzhou, Guangdong 510005 (CN); CHEN, Caiyan, Guangzhou, Guangdong 510005 (CN); LIN, Qixing, Guangzhou, Guangdong 510005 (CN); LI, Chuang, Guangzhou, Guangdong 510005 (CN); ZHANG, Hui, Guangzhou, Guangdong 510005 (CN); LI, Jiaping, Guangzhou, Guangdong 510005 (CN); CHEN, Junyou, Guangzhou, Guangdong 510005 (CN); WANG, Zhiwei, Guangzhou, Guangdong 510005 (CN); HUANG, Xianming, Guangzhou, Guangdong 510005 (CN)
(74) Representative: Engelhard, Markus
(86) International application number: PCT/CN2024/102562
(87) International publication number: WO 2025/002405

(57) **Abstract**

Provided is a 4-1BB-binding protein that can specifically bind to 4-1BB and does not block the binding of 4-1BB to 4-1BBL. The protein can effectively activate NF-κB signaling and T cells under cross-linking conditions.

## Description

### TECHNICAL FIELD

The present disclosure pertains to the field of bio-pharmaceuticals and relates to a 4-1BB-binding protein, such as a single-domain antibody, a heavy-chain antibody, and/or a fusion protein binding to 4-1BB, and use thereof.

### BACKGROUND

4-1BB, also known as CD137, belongs to the tumor necrosis factor receptor superfamily and plays an important role in T cell activation-driven immune responses. It mainly promotes T cell proliferation, enables T cells to acquire effector functions, promotes immune memory, and inhibits activation-induced cell death. The earliest 4-1BB-targeting agonistic antibodies to enter clinical trials include urelumab, developed by Bristol-Myers Squibb (BMS), and utomilumab, developed by Pfizer. They have shown potential as monotherapies or a combination therapy in mouse tumor models. However, due to toxicity or lack of efficacy in clinical cases (Chester C, Sanmamed MF, Wang J, Melero I. Immunotherapy targeting 4-1BB: mechanistic rationale, clinical results, and future strategies. Blood. 2018;131(1):49-57.), there is a great clinical need for safe and effective 4-1BB-targeting novel therapies.

### SUMMARY

The present disclosure provides a 4-1BB-binding protein that can specifically bind to 4-1BB and does not block the binding of 4-1BB to 4-1BBL (a 4-1BB ligand). The protein can effectively activate NF-κB signaling and T cells under cross-linking conditions.

In one aspect, the present disclosure provides a 4-1BB-binding protein, comprising a heavy chain variable region, wherein the heavy chain variable region comprises one or more of the following complementarity determining regions (CDRs): an HCDR1 set forth in SEQ ID NO: 7, an HCDR2 set forth in any one of SEQ ID NOs: 8-13 or SEQ ID NOs: 31-36, and an HCDR3 set forth in any one of SEQ ID NOs: 14-19 or SEQ ID NOs: 37-42.

In some embodiments, the heavy chain variable region comprises an HCDR1 set forth in SEQ ID NO: 7, an HCDR2 set forth in SEQ ID NOs: 8-13, and an HCDR3 set forth in any one of SEQ ID NOs: 14-19.

In some embodiments, the heavy chain variable region comprises an HCDR1 set forth in SEQ ID NO: 7, an HCDR2 set forth in SEQ ID NO: 8, and an HCDR3 set forth in any one of SEQ ID NOs: 14-17.

In some embodiments, the heavy chain variable region comprises an HCDR1 set forth in SEQ ID NO: 7, an HCDR2 set forth in SEQ ID NO: 9, and an HCDR3 set forth in SEQ ID NO: 14 or 19.

In some embodiments, the heavy chain variable region comprises an HCDR1 set forth in SEQ ID NO: 7, an HCDR2 set forth in SEQ ID NO: 10, and an HCDR3 set forth in SEQ ID NO: 14 or 16.

In some embodiments, the heavy chain variable region comprises an HCDR1 set forth in SEQ ID NO: 7, an HCDR2 set forth in SEQ ID NO: 11 or 12, and an HCDR3 set forth in SEQ ID NO: 16.

In some embodiments, the heavy chain variable region comprises an HCDR1 set forth in SEQ ID NO: 7, an HCDR2 set forth in SEQ ID NO: 13, and an HCDR3 set forth in SEQ ID NO: 18 or 19.

In some embodiments, the heavy chain variable region comprises an HCDR1 set forth in SEQ ID NO: 7, an HCDR2 set forth in SEQ ID NOs: 31-36, and an HCDR3 set forth in any one of SEQ ID NOs: 37-42.

In some embodiments, the heavy chain variable region comprises an HCDR1 set forth in SEQ ID NO: 7, an HCDR2 set forth in SEQ ID NO: 31, and an HCDR3 set forth in any one of SEQ ID NOs: 37-40.

In some embodiments, the heavy chain variable region comprises an HCDR1 set forth in SEQ ID NO: 7, an HCDR2 set forth in SEQ ID NO: 32, and an HCDR3 set forth in SEQ ID NO: 37 or 42.

In some embodiments, the heavy chain variable region comprises an HCDR1 set forth in SEQ ID NO: 7, an HCDR2 set forth in SEQ ID NO: 33, and an HCDR3 set forth in SEQ ID NO: 37 or 39.

In some embodiments, the heavy chain variable region comprises an HCDR1 set forth in SEQ ID NO: 7, an HCDR2 set forth in SEQ ID NO: 34 or 35, and an HCDR3 set forth in SEQ ID NO: 39.

In some embodiments, the heavy chain variable region comprises an HCDR1 set forth in SEQ ID NO: 7, an HCDR2 set forth in SEQ ID NO: 36, and an HCDR3 set forth in SEQ ID NO: 41 or 42.

In some embodiments, the heavy chain variable region comprises an amino acid sequence set forth in any one of SEQ ID NOs: 1-6 and 29-30, or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity compared to the amino acid sequence set forth in any one of SEQ ID NOs: 1-6 and 29-30, or an amino acid sequence having one or more conservative amino acid substitutions compared to the amino acid sequence set forth in any one of SEQ ID NOs: 1-6 and 29-30.

In some embodiments, the heavy chain variable region comprises an amino acid sequence set forth in any one of SEQ ID NOs: 1-6 and 29-30.

In some embodiments, the 4-1BB-binding protein is an antibody or an antigen-binding fragment. In some embodiments, the 4-1BB-binding protein is in the form of a single-domain antibody (e.g., a nanobody), a heavy-chain antibody, a bispecific antibody, a multispecific antibody, a Fab, a Fab', a F(ab')2, an Fv, or an scFv.

In some embodiments, the 4-1BB-binding protein is a fusion protein further comprising an Fc region. In some embodiments, the Fc is an Fc of an immunoglobulin. In some embodiments, the Fc is an Fc of an IgG. In some embodiments, the Fc is an Fc ofIgG 1, IgG2, IgG3, or IgG4, or a variant thereof. In some embodiments, an amino acid sequence of the Fc of IgG1 is set forth in SEQ ID NO: 21.

In some embodiments, the Fc is a variant Fc region. In some embodiments, the variant Fc region has one or more amino acid modifications, such as substitutions, deletions, or insertions, compared to the parent Fc region. In some embodiments, the amino acid modifications of the Fc region alter effector function activity, compared to the activity of the parent Fc region. In some embodiments, the variant Fc region can have altered (i.e., increased or decreased) antibody-dependent cellular cytotoxicity (ADCC), complement-dependent cytotoxicity (CDC), phagocytosis, opsonization, or cell binding activity. In some embodiments, the amino acid modifications of the Fc region can alter the affinity for an FcγR (Fcγ receptor), compared to the parent Fc region. In some embodiments, the Fc region is derived from IgG1 or IgG4. In some embodiments, the Fc mutation comprises one or more of mutations N297A, L234A/F234A, L235A, L235E, and G237A (Eu numbering). In some embodiments, the Fc region mutation is E345R or S440Y (Eu numbering). In some embodiments, the IgG1 Fc comprises mutation N297A (Eu numbering).

In some embodiments, in the fusion protein, the C-terminus of the heavy chain variable region and the N-terminus of the Fc region are further linked by a peptide linker. In some embodiments, a sequence of the peptide linker is (GₘS)ₙ, wherein each m is independently 2, 3, 4, or 5, and n is independently 1, 2, 3, 4, 5, or 6. In some embodiments, a sequence of the linker is (GGGGS)ₙ, wherein n is independently 1, 2, 3, 4, 5, or 6. In some embodiments, an amino acid sequence of the peptide linker 1 is set forth in SEQ ID NO: 8.

In some embodiments, the fusion protein comprises an amino acid sequence set forth in any one of SEQ ID NOs: 22-27, or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity compared to the amino acid sequence set forth in any one of SEQ ID NOs: 22-27, or an amino acid sequence having one or more conservative amino acid substitutions compared to the amino acid sequence set forth in any one of SEQ ID NOs: 22-27.

In some embodiments, the fusion protein comprises the amino acid sequence set forth in any one of SEQ ID NOs: 22-27.

In some embodiments, the fusion protein comprises two identical amino acid sequences.

**Table 1: CDR amino acid sequences**

| SEQ ID NO. | HCDR1 | SEQ ID NO. | HCDR2 | SEQ ID NO. | HCDR3 |
|---|---|---|---|---|---|
| | | 8 | GNGGSTY | 14 | GYSYLYPGGLSTRPSFY |
| | | 8 | GNGGSTY | 15 | GDAYVYPYGVSTDPSFY |
| | | 9 | GSGGSTY | 14 | GYSYLYPGGLSTRPSFY |
| | | 10 | GNGGSTF | 14 | GYSYLYPGGLSTRPSFY |
| | | 11 | GYGGTTY | 16 | GTGYYSRVGVSTDPSFY |
| | | 8 | GNGGSTY | 17 | GYSYLSPGGVSTRPSFY |
| | | 12 | GSGGTTY | 16 | GTGYYSRVGVSTDPSFY |
| | | 13 | GYGGSTY | 18 | GSGLYSSYGVSTDPSFY |
| | | 10 | GNGGSTF | 16 | GTGYYSRVGVSTDPSFY |
| | | 9 | GSGGSTY | 19 | GSAYGYSGGLSTDPSFY |
| | | 13 | GYGGSTY | 19 | GSAYGYSGGLSTDPSFY |
| 7 | SYAMG | 8 | GNGGSTY | 16 | GTGYYSRVGVSTDPSFY |
| | | 31 | AISGNGGST YYADSVKG | 37 | GYSYLYPGGLSTRPSFYDY |
| | | 32 | AISGSGGST YYADSVKG | 38 | GDAYVYPYGVSTDPSFYDY |
| | | 33 | AISGNGGST FYADSVKG | 39 | GTGYYSRVGVSTDPSFYDY |
| | | 34 | AISGYGGTT YYADSVKG | 40 | GYSYLSPGGVSTRPSFYDY |
| | | 35 | AISGSGGTT YYADSVKG | 41 | GSGLYSSYGVSTDPSFYDY |
| | | 36 | AISGYGGST YYADSVKG | 42 | GSAYGYSGGLSTDPSFYDY |

**Table 2: Amino acid sequences**

| SEQ ID NO. | Amino acid sequence |
|---|---|
| 1 | EVQLVESGGGLVQPGGSLRLSCAASGFTFSSYAMGWVRQAPGKGREFVS AISGNGGSTYYADSVKGRFTISRDNSKNTLYLQMNSLRAEDTAVYYCAAG YSYLYPGGLSTRPSFYDYWGQGTQVTVSS |
| 2 | EVQLVESGGGLVQPGGSLRLSCAASGFTFSSYAMGWVRQAPGKGREFVS AISGNGGSTYYADSVKGRFTISRDNSKNTLYLQMNSLRAEDTAVYYCAAG DAYVYPYGVSTDPSFYDYWGQGTQVTVSS |
| 3 | EVQLVESGGGLVQPGGSLRLSCAASGFTFSSYAMGWVRQAPGKGREFVS AISGSGGSTYYADSVKGRFTISRDNSKNTLYLQMNSLRAEDTAVYYCAAG YSYLYPGGLSTRPSFYDYWGQGTQVTVSS |
| 4 | EVQLVESGGGLVQPGGSLRLSCAASGFTFSSYAMGWVRQAPGKGREFVS AISGNGGSTFYADSVKGRFTISRDNSKNTLYLQMNSLRAEDTAVYYCAAG YSYLYPGGLSTRPSFYDYWGQGTQVTVSS |
| 5 | EVQLVESGGGLVQPGGSLRLSCAASGFTFSSYAMGWVRQAPGKGREFVS AISGYGGTTYYADSVKGRFTISRDNSKNTLYLQMNSLRAEDTAVYYCAAG TGYYSRVGVSTDPSFYDYWGQGTQVTVSS |
| 6 | EVQLVESGGGLVQPGGSLRLSCAASGFTFSSYAMGWVRQAPGKGREFVS AISGYGGSTYYADSVKGRFTISRDNSKNTLYLQMNSLRAEDTAVYYCAAG SGLYSSYGVSTDPSFYDYWGQGTQVTVSS |
| 20 | *GGGGSGGGGS* |
| 21 | EPKSSDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLT CLVKGFYPSDIAVEWESNGQPENNYKTTPPVLDSDGSFFLYSKLTVDKSR WQQGNVFSCSVMHEALHNHYTQKSLSLSPGK |
| 22 | EVQLVESGGGLVQPGGSLRLSCAASGFTFSSYAMGWVRQAPGKGREFVS AISGNGGSTYYADSVKGRFTISRDNSKNTLYLQMNSLRAEDTAVYYCAAG YSYLYPGGLSTRPSFYDYWGQGTQVTVSSGGGGSGGGGSEPKSSDKTHT CPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSC SVMHEALHNHYTQKSLSLSPGK |
| 23 | EVQLVESGGGLVQPGGSLRLSCAASGFTFSSYAMGWVRQAPGKGREFVS AISGNGGSTYYADSVKGRFTISRDNSKNTLYLQMNSLRAEDTAVYYCAAG DAYVYPYGVSTDPSFYDYWGQGTQVTVSSGGGGSGGGGSEPKSSDKTHT CPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSC SVMHEALHNHYTQKSLSLSPGK |
| 24 | EVQLVESGGGLVQPGGSLRLSCAASGFTFSSYAMGWVRQAPGKGREFVS AISGSGGSTYYADSVKGRFTISRDNSKNTLYLQMNSLRAEDTAVYYCAAG YSYLYPGGLSTRPSFYDYWGQGTQVTVSSGGGGSGGGGSEPKSSDKTHT CPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSC SVMHEALHNHYTQKSLSLSPGK |
| 25 | EVQLVESGGGLVQPGGSLRLSCAASGFTFSSYAMGWVRQAPGKGREFVS AISGNGGSTFYADSVKGRFTISRDNSKNTLYLQMNSLRAEDTAVYYCAAG YSYLYPGGLSTRPSFYDYWGQGTQVTVSSGGGGSGGGGSEPKSSDKTHT CPPCPAPELLGGPSVFLFPPKPKDTLMISRTPEVTCVVVDVSHEDPEVKFN |
| | WYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSC SVMHEALHNHYTQKSLSLSPGK |
| 26 | EVQLVESGGGLVQPGGSLRLSCAASGFTFSSYAMGWVRQAPGKGREFVS AISGYGGTTYYADSVKGRFTISRDNSKNTLYLQMNSLRAEDTAVYYCAAG TGYYSRVGVSTDPSFYDYWGQGTQVTVSSGGGGSGGGGSEPKSSDKTHT CPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSC SVMHEALHNHYTQKSLSLSPGK |
| 27 | EVQLVESGGGLVQPGGSLRLSCAASGFTFSSYAMGWVRQAPGKGREFVS AISGYGGSTYYADSVKGRFTISRDNSKNTLYLQMNSLRAEDTAVYYCAAG SGLYSSYGVSTDPSFYDYWGQGTQVTVSSGGGGSGGGGSEPKSSDKTHT CPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSC SVMHEALHNHYTQKSLSLSPGK |
| 28 | MGNSCYNIVATLLLVLNFERTRSLQDPCSNCPAGTFCDNNRNQICSPCPPN SFSSAGGQRTCDICRQCKGVFRTRKECSSTSNAECDCTPGFHCLGAGCSM CEQDCKQGQELTKKGCKDCCFGTFNDQKRGICRPWTNCSLDGKSVLVNG TKERDVVCGPSPADLSPGASSVTPPAPAREPGHSPQIISFFLALTSTALLFLL FFLTLRFSVVKRGRKKLLYIFKQPFMRPVQTTQEEDGCSCRFPEEEEGGCE L |
| 29 | EVQLVESGGGLVQPGGSLRLSCAASGFTFSSYAMGWVRQAPGKGREFVS AISGNGGSTYYADSVKGRFTISRDNSKNTLYLQMNSLRAEDTAVYYCAAG YSYLSPGGVSTRPSFYDYWGQGTQVTVSS |
| 30 | EVQLVESGGGLVQPGGSLRLSCAASGFTFSSYAMGWVRQAPGKGREFVS AISGSGGTTYYADSVKGRFTISRDNSKNTLYLQMNSLRAEDTAVYYCAAG TGYYSRVGVSTDPSFYDYWGQGTQVTVSS |

In one aspect, the present disclosure provides a biomaterial that is:
1) a nucleic acid molecule encoding the 4-1BB-binding protein of the present disclosure or a portion thereof;
2) an expression vector comprising the nucleic acid molecule of the present disclosure; or
3) a host cell comprising the nucleic acid molecule or the expression vector of the present disclosure.

In one aspect, the present disclosure provides a pharmaceutical composition comprising the 4-1BB-binding protein of the present disclosure and a pharmaceutically acceptable carrier.

In one aspect, the present disclosure provides use of the 4-1BB-binding protein, the biomaterial, or the pharmaceutical composition described above in the preparation of a medicament for treating or preventing a disease.

In one aspect, the present disclosure provides use of the 4-1BB-binding protein, the biomaterial, or the pharmaceutical composition described above in the treatment or prevention of a disease. In one aspect, the present disclosure provides a method for treating or preventing a disease, comprising administering to a patient in need thereof an effective dose of the 4-1BB-binding protein, the biomaterial, or the pharmaceutical composition described above. In some embodiments, the disease is a tumor, a viral infection, an autoimmune disease, or an inflammatory disease. In some embodiments, the tumor includes melanoma, non-small cell lung cancer, small cell lung cancer, head and neck cancer, liver cancer, colon cancer, prostate cancer, gastric cancer, renal cancer, bladder cancer, pancreatic cancer, breast cancer, ovarian cancer, endometrial cancer, esophageal cancer, soft tissue sarcoma, bile duct cancer, thyroid cancer, hepatocellular carcinoma, mesothelioma, etc.

### BRIEF DESCRIPTION OF THE DRAWINGS

FIG. 1: The results of a binding experiment of 4-1BB-VHH-Fc fusion proteins to 4-1BB-expressing cells.
FIGs. 2 and 3: A binding competition experiment of 4-1BB-VHH-Fc fusion proteins to the 4-1BB ligand.
FIG. 4: A comparison of the activation of the 293T-41BB reporter system by different fusion proteins in the presence of a cross-linking antibody, where the cross-linking antibody is an anti-human IgG (Fc-specific).
FIG. 5: A comparison of the activation of the reporter system after immobilization and cross-linking of different fusion proteins to a 96-well plate.

### DETAILED DESCRIPTION

Unless otherwise defined, all technical and scientific terms used herein have the same meanings as those commonly understood by those of ordinary skill in the art to which the present disclosure pertains.

### Terms

It should be noted that the term "an" entity refers to one or more of the entities. For example, "an antibody" should be interpreted as one or more antibodies. As such, the terms "a" (or "an"), "one or more", and "at least one" are used interchangeably herein. As used herein, the term "comprise", "contain", or "include" means that the antibodies, compositions, methods, or the like include the recited elements, such as components or steps, and do not exclude the others. "Consisting essentially of..." means that the antibodies, compositions, methods, or the like exclude other elements that have a fundamental impact on the characteristics of the combination, but do not exclude elements that do not substantially affect the antibodies, compositions, methods, or the like. "Consisting of..." means that elements not specifically recited are excluded. The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, the definition of "polypeptide" includes peptides, dipeptides, tripeptides, oligopeptides, "proteins", "amino acid chains", or any other terms used to refer to chains of two or more amino acids, and "polypeptide" may be used in place of, or interchangeably with, any one of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modifications. A polypeptide may be derived from a natural biological source or produced by recombinant techniques, but it is not necessarily translated from a specified nucleic acid sequence. It may be produced in any manner, including chemical synthesis.

In the present disclosure, the 4-1BB antigen may be derived from a mammal, such as a human, a rat, a mouse, or a monkey.

It will be appreciated by those of ordinary skill in the art that the CDRs of an antibody are responsible for the binding specificity of the antibody to an antigen. Where the heavy and light chain variable region sequences of an antibody are known, there are currently several methods for determining the CDRs of the antibody, including the Kabat, IMGT, Chothia, and AbM numbering systems. However, every application of the definition of CDRs regarding an antibody or a variant thereof will fall within the scope of the terms defined and used herein. If the amino acid sequences of the variable regions of the antibody are given, those skilled in the art can generally determine which residues are included in particular CDRs, without relying on any experimental data other than the sequences.

"Antibody" or "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising in the molecule at least a portion of an immunoglobulin molecule that has biological activity for binding to an antigen. Antibodies and antigen-binding fragments include, but are not limited to, complementarity determining regions (CDRs) of heavy or light chains or ligand-binding portions thereof, heavy chain variable regions (VHs), light chain variable regions (VLs), heavy chain constant regions (CHs), light chain constant regions (CLs), framework regions (FRs) or any portion thereof, or at least a portion of a binding protein. CDRs include light chain CDRs (LCDR1-3) and heavy chain CDRs (HCDR1-3). A heavy chain constant region (CH) comprises a CH1 domain, a hinge (e.g., an upper hinge region, a middle hinge region, and/or a lower hinge region) domain, a CH2 domain, and a CH3 domain. A crystallizable segment (Fc region) is equivalent to the CH2 and CH3 domains. An antibody or antigen-binding fragment can specifically recognize and bind to one or more (e.g., two) polypeptides or polypeptide complexes of antigens. An antibody or antigen-binding fragment that specifically recognizes and binds to multiple (e.g., two) antigens can be referred to as a multispecific (e.g., bispecific) antibody or antigen-binding fragment.

An intact antibody comprises a heavy chain and a light chain. Classes of heavy chains include gamma, mu, alpha, delta, or epsilon (γ, µ, α, δ, or ε), including some subclasses (e.g., γ1-γ4). The nature of this chain determines that the "type" of the antibody is IgG, IgM, IgA, IgD, or IgE. Antibody subclasses (isotypes), such as IgG1, IgG2, IgG3, and IgG4, have been well characterized, and the functional specificity imparted is also known. All types of antibodies fall within the protection scope of the present disclosure. In some embodiments, an antibody comprises two heavy chains or/and two light chains, and the four chains are linked by disulfide bonds to form a "Y" configuration, wherein the light chains start and extend from the opening of "Y" and surround the heavy chains through variable regions.

The term "antibody fragment" or "antigen-binding fragment" refers to a portion of an antibody, such as F(ab')2, F(ab)2, Fab', Fab, Fv, or scFv. Regardless of its structure, an antibody fragment binds to the same antigen recognized by an intact antibody. The term "antibody fragment" includes aptamers, mirror image isomers, and bivalent antibodies. The term "antigen-binding fragment" also includes any synthetic or genetically engineered protein that functions as an antibody by binding to a specific antigen to form a complex.

The term "Fab" generally refers to the portion of a conventional antibody (e.g., IgG) that binds to an antigen, including the heavy chain variable region VH, the light chain variable region VL, the heavy chain constant region domain CH1, and the light chain constant region CL of the antibody. In a conventional antibody, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In some examples, "Fab" also refers to a variant structure of Fab. For example, in certain examples, the C-terminus of VH is linked to the N-terminus of CL to form one polypeptide chain, and the C-terminus of VL is linked to the N-terminus of CH1 to form another polypeptide chain, in which case an Fab (cross VH/VL) structure is formed; in certain examples, CH1 of Fab is not linked to the hinge region, while the C-terminus of CL is linked to the hinge region of the heavy chain, in which case a Fab (cross Fd/LC) structure is formed.

"Single-chain antibody", "single-chain variable fragment", or "scFv" refers to a fusion protein of a heavy chain variable region (VH) and a light chain variable region (VL) of an immunoglobulin. In some aspects, these regions are linked to a short linker peptide of 10 to about 25 amino acids. The linker may be enriched with glycine to improve flexibility, and enriched with serine or threonine to improve solubility, and may link the N-terminus of VH and the C-terminus of VL, or vice versa. Although the constant regions are removed from the protein and a linker is introduced into the protein, it retains the specificity of the original immunoglobulin. scFv molecules are generally known in the art and are described in, for example, U.S. Patent No. 5,892,019.

The term "single-domain antibody" or "sdAb" refers to an antigen-binding fragment that comprises only a single antibody variable region. An individual sdAb is capable of binding to an antigen, but need not be paired with a corresponding CDR-containing polypeptide. In some cases, an sdAb is obtained by engineering a camelid HcAb, and the heavy chain variable domain thereof is referred to herein as "VHH" (the heavy chain variable domain of a heavy-chain antibody). Some VHHs are also referred to as nanobodies. From the N-terminus to the C-terminus, a VHH has the following structure: FR1-CDR1-FR2-CDR2-FR3-CDR3-FR4. Herein, a VHH that can bind to 4-1BB may be referred to as a 4-1BB-VHH.

The term "heavy-chain antibody" or "HcAb" refers to a functional antibody that comprises heavy chains (VHH, CH2, and CH3) but lacks the light chains typically present in antibodies. Camelid animals (e.g., camels, llamas, or alpacas) are known to produce HcAbs.

"Homology", "identity", or "similarity" refers to sequence similarity between two peptides or between two nucleic acid molecules. Homology or identity can be determined by comparing the positions that can be aligned in the sequences. When a position of the compared sequences is occupied by the same base or amino acid, the molecules are homologous or identical at that position. The degree of homology between sequences is a function of the number of matching or homologous positions shared by the sequences.

A polynucleotide or a polynucleotide sequence (or a polypeptide or an antibody sequence) having a certain percentage (e.g., 90%, 95%, 98%, or 99%) of "identity or sequence identity" to another sequence means that when the sequences are aligned, the percentage of bases (or amino acids) in the two compared sequences are the same. This alignment and identity percentage or sequence identity can be determined using visual inspection or software programs known in the art, such as the software programs described in Ausubel et al. eds., (2007), Current Protocols in Molecular Biology*.* Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGHSCORE; Databases = non-redundant; GenBank + EMBL + DDBJ + PDB + GenBank CDS translations + Swiss Protein + SPupdate + PIR. Biologically equivalent polynucleotides are polynucleotides having the above-specified percentage identity and encoding polypeptides having identical or similar biological activity.

A "conservative amino acid substitution" is the substitution of an amino acid residue with an amino acid residue having a similar side chain. Families of amino acid residues having similar side chains have been defined in the art, including basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, and tryptophan), β-branched side chains (e.g., threonine, valine, and isoleucine), and aromatic side chains (e.g., tyrosine, phenylalanine, tryptophan, and histidine). Thus, non-essential amino acid residues of an immunoglobulin polypeptide are preferably substituted with other amino acid residues from the same side chain family. In some other embodiments, a series of amino acids may be substituted with a series of structurally similar amino acids that differ in sequence and/or composition of the side chain family.

In some embodiments, the conservative substitution is preferably the substitution of one amino acid within the following groups (a)-(e) with another amino acid residue within the same group: (a) small aliphatic, non-polar or weakly polar residues: Ala, Ser, Thr, Pro, and Gly; (b) polar, negatively charged residues and their (uncharged) amides: Asp, Asn, Glu, and Gln; (c) polar, positively charged residues: His, Arg, and Lys; (d) large aliphatic, nonpolar residues: Met, Leu, Ile, Val, and Cys; and (e) aromatic residues: Phe, Tyr, and Trp.

Particularly preferred conservative substitutions are as follows: substitution of Ala with Gly or Ser; substitution of Arg with Lys; substitution of Asn with Gln or His; substitution of Asp with Glu; substitution of Cys with Ser; substitution of Gln with Asn; substitution of Glu with Asp; substitution of Gly with Ala or Pro; substitution of His with Asn or Gln; substitution of Ile with Leu or Val; substitution of Leu with Ile or Val; substitution of Lys with Arg, Gln, or Glu; substitution of Met with Leu, Tyr, or Ile; substitution of Phe with Met, Leu, or Tyr; substitution of Ser with Thr; substitution of Thr with Ser; substitution of Trp with Tyr; substitution of Tyr with Trp; and/or substitution of Phe with Val, Ile, or Leu.

The 4-1BB-binding protein disclosed by the present disclosure includes modified derivatives, i.e., those that are modified by covalent linking of any type of molecule to the 4-1BB-binding protein, wherein the covalent linking does not prevent the 4-1BB-binding protein from binding to an epitope. The 4-1BB-binding protein may be subjected to glycosylation, acetylation, PEGylation, phosphorylation, amidation, derivatization by known protecting/blocking groups, proteolytic cleavage, attachment to cellular ligands or other proteins, or the like. Any one of various chemical modifications may be made by techniques in the prior art, including but not limited to specific chemical cleavage, acetylation, formylation, metabolic synthesis of tunicamycin, etc.

In some embodiments, the 4-1 BB-binding protein may be conjugated with a therapeutic agent, a prodrug, a peptide, a protein, an enzyme, a virus, a lipid, a biological response modifier, an agent, or PEG.

"Pharmaceutically acceptable" refers to materials listed in the pharmacopeia for use in drugs for animals, particularly for humans. In addition, "pharmaceutically acceptable carrier and/or excipient" will generally be any type of non-toxic solid, semi-solid, or liquid filler, diluent, encapsulating material, or formulation additive.

The term "carrier" refers to a diluent, adjuvant, excipient, or carrier that can be administered to a patient together with the active ingredient. Such pharmaceutical carriers may be sterile liquids, such as water and oils, including oils derived from petroleum, animals, and plants or synthetic oils, such as peanut oil, soybean oil, mineral oil, and sesame oil. Water is a preferred carrier when the pharmaceutical composition is administered intravenously. A saline aqueous solution, a glucose aqueous solution, and a glycerol solution may also be used as a liquid carrier, especially for injection. Suitable pharmaceutical excipients include starch, gelatin, malt, rice, flour, chalk, silica gel, sodium stearate, glycerol monostearate, talc, skimmed milk powder, glycerol, propene, ethylene glycol, water, ethanol, etc. If desired, the composition may also contain a small amount of a wetting agent, an emulsifier, or a pH buffering agent. Antibacterials such as benzyl alcohol or methylparaben and antioxidants such as ascorbic acid or sodium bisulfite are also contemplated. These compositions may be in the form of solutions, suspensions, emulsions, tablets, pills, capsules, powders, sustained-release formulations, etc. The composition may be formulated as a suppository using conventional binders and carriers such as triglyceride. Oral formulations may comprise standard carriers such as mannitol, lactose, starch, magnesium stearate, sodium saccharin, cellulose, and magnesium carbonate of pharmaceutical grade. Examples of suitable pharmaceutical carriers are described in Remington's Pharmaceutical Sciences by E. W. Martin, which is incorporated herein by reference. Such compositions will contain a clinically effective dose of an antibody or an antigen-binding fragment, preferably in a purified form, together with an appropriate amount of a carrier and/or excipient, to provide a form suitable for administration to a patient. The formulation should be suitable for the administration mode. The formulation may be encapsulated in an ampoule bottle, a disposable syringe, or a multi-dose vial made of glass or plastic.

The 4-1BB-binding protein (including antibodies, antigen-binding fragments, fusion proteins, or the like) of the present disclosure includes neutral or salt forms. The pharmaceutically acceptable salts include, but are not limited to, salts formed with anions derived from acids such as hydrochloric acid, phosphoric acid, acetic acid, oxalic acid, and tartaric acid, and salts formed with cations derived from, e.g., sodium, potassium, ammonium, calcium, iron hydroxide, isopropylamine, triethylamine, 2-ethylaminoethanol, histidine, and procaine. The 4-1BB-binding protein described herein may be neutral, i.e., having substantially no net charge; for example, when the pH of the composition comprising the 4-1BB-binding protein is the isoelectric point of the protein, the protein is electrically neutral. The protein described herein may be present in the form of a positive ion; for example, when the pH is below the isoelectric point, the protein molecule as a whole displays a positive charge. The protein described herein may be present in the form of a negative ion; for example, when the pH is above the isoelectric point, the protein molecule as a whole displays a negative charge.

The effective dosage and therapeutic regimen for treating a particular patient will depend upon a variety of factors, including the particular 4-1BB-binding protein or derivative used, the age, body weight, general health condition, sex, and diet of the patient, and the time of administration, frequency of excretion, drug combination, and the severity of the particular disease being treated. These factors are judged by medical caregivers who fall within the scope of those of ordinary skill in the art. The dose employed can be determined by pharmacological and pharmacokinetic principles well known in the art. In some embodiments, the 4-1BB-binding protein of the present disclosure is administered to the patient at a dose of 0.01 mg/kg to 100 mg/kg body weight. In some embodiments, administration is performed once every week, every two weeks, every three weeks, or every four weeks.

"About" refers to a conventional margin of error for corresponding values as readily understood by those skilled in the relevant art. In some embodiments, the term "about" mentioned herein refers to the values described and a range of ±10%, ±5%, or ±1% thereof.

### Preparation of Antibodies or Fusion Proteins

A variety of methods for preparing antibodies or fusion proteins are known in the art. Methods such as hybridoma techniques, recombinant DNA techniques, transgenic mouse techniques, and phage display libraries are used for preparing antibodies.

Antibodies or fusion proteins can be prepared by using conventional recombinant DNA techniques. Vectors and cell lines for antibody or fusion protein production can be selected, constructed, and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated herein by reference in its entirety, including the supplements.

In some embodiments, the DNA encoding the antibody or the fusion protein can be designed and synthesized according to the amino acid sequence of the antibody or the fusion protein described herein by conventional methods and inserted into an expression vector, and a host cell is then transfected with the expression vector. The transfected host cell is cultured in a culture medium to produce the monoclonal antibody or the fusion protein. In some embodiments, the expression vector comprises at least one promoter element, a protein-encoding sequence, a transcription termination signal, and a polyA tail. Other elements include enhancers, Kozak sequences, and donor and recipient sites flanking the inserted sequence for RNA splicing. Efficient transcription can be achieved by early and late promoters of SV40, long terminal repeats from retroviruses such as RSV, HTLV1, and HIVI, and early promoters of cytomegalovirus, and promoters from other cells, such as actin promoters, can also be used. Suitable expression vectors may include pIRES1neo, pRetro-Off, pRetro-On, PLXSN, pLNCX, pcDNA3.1 (+/-), pcDNA/Zeo (+/-), pcDNA3.1/Hygro (+/-), PSVL, PMSG, pRSVcat, pSV2dhfr, pBC12MI, pCS2, etc. Commonly used mammalian host cells include HEK293 cells, Cos1 cells, Cos7 cells, CV1 cells, murine L cells, CHO cells, etc.

In some embodiments, the inserted gene fragment should comprise a screening label (common screening labels include screening genes such as dihydrofolate reductase, glutamine synthetase, neomycin resistance, and hygromycin resistance) to facilitate the screening and separation of cells that have been successfully transfected. Host cells without the above genes are transfected with the constructed plasmid. Through culture in a selective culture medium, the successfully transfected cells grow extensively and produce the desired target protein. The resulting antibody or fusion protein can be isolated or purified by conventional technical approaches, such as protein A-sepharose, ion exchange chromatography, hydroxyapatite chromatography, gel electrophoresis, or affinity chromatography.

### Example 1: Enrichment and Screening of Anti-4-1BB VHH Positive Clones

From a phage display library, 4-1BB-positive VHH clones were enriched by biopanning using a biotinylated 4-1BB antigen, and 192 single clones were identified. Then VHHs with binding activity to the human 4-1BB antigen were selected by ELISA. The positive clones were sequenced, and their CDR sequences were compared and analyzed. Their sequences and the contained CDRs are shown in Table 3. The amino acid sequences are shown in Table 1 and Table 2.

**Table 3: The composition of 4-1BB VHHs**

| Name | Amino acid sequence | HCDR1 | HCDR2 | HCDR3 |
|---|---|---|---|---|
| VHH1 | 1 | 7 | 8 | 14 |
| VHH2 | 2 | | 8 | 15 |
| VHH6 | 3 | | 9 | 14 |
| VHH8 | 4 | | 10 | 14 |
| VHH13 | 5 | | 11 | 16 |
| VHH15-1 | 29 | | 8 | 17 |
| VHH16 | 30 | | 12 | 16 |
| VHH15 | 6 | | 13 | 18 |

### Example 2: Construction, Expression, and Biophysical Characterization of 4-1BB-VHH-Fc Fusion Proteins

This example describes the construction and expression of exemplary 4-1BB-VHH-Fc fusion proteins. Seven fusion proteins were designed and expressed, and each of them contained, from the N-terminus to the C-terminus, one polypeptide chain of the following structure: the VHH domain (whose sequence is set forth in SEQ ID NOs: 1-6) of an anti-4-1BB single-domain antibody (sdAb)-a peptide linker (linker 1, whose sequence is set forth in SEQ ID NO: 20)-the Fc domain of IgG1 (whose sequence is set forth in SEQ ID NO: 21). The specific names and corresponding sequences are shown in Table 4.

**Table 4: The structures and corresponding sequences of 4-1BB-VHH-Fc fusion proteins**

| Fusion protein name | 4-1BB-VHH (SEQ ID NO:) | Linker 1 (SEQ ID NO:) | Fc (SEQ ID NO:) |
|---|---|---|---|
| VHH1-Fc | 1 | 20 | 21 |
| VHH2-Fc | 2 | 20 | 21 |
| VHH6-Fc | 3 | 20 | 21 |
| VHH8-Fc | 4 | 20 | 21 |
| VHH13-Fc | 5 | 20 | 21 |
| VHH15-Fc | 6 | 20 | 21 |

The positive control antibodies were urelumab and utomilumab.

Plasmids expressing the above fusion proteins and the two positive control antibodies were prepared and transiently expressed in 293F cells. The products were purified by protein A. According to sequencing analysis, the sequences of the 8 proteins were consistent with the designs.

### Example 3: Binding Assay of 4-1BB-VHH-Fc Fusion Proteins to Target

The ability of fusion proteins to bind to 4-1BB can be determined by surface plasmon resonance (e.g., Biacore), fluorescence-assisted cell sorting (FACS), or a combination thereof. Analysis can be performed on activated T cells.

The binding kinetics of different fusion proteins to 4-1BB were determined using the surface plasmon resonance (SPR) biosensor BIACORE T200 (GE Healthcare). 4-1BB-VHH-Fc fusion protein samples with different concentrations were prepared by 3-fold serial dilution starting from 100 nM with a HEPES-buffered saline buffer for BIAcore. A total of 7 gradients were obtained. Each sample was fixed to the sensor chip by the Fc capture method. The antigen 4-1BB (Sino Biological, Cat. No. 10041-H08H) was used as the analyte. Dissociation (kd) and association (ka) rate constants were obtained using T200 evaluation software (Biacore, GE Healthcare). The apparent equilibrium dissociation constant (K_{D}) was calculated using the ratio of K_{d} to Kₐ. As shown in Table 5, the fusion proteins were comparable to the positive control antibodies in terms of 4-1BB-binding kinetics.

**Table 5: The affinities of 4-1BB-VHH-Fc fusion proteins**

| | ka (1/Ms) | kd (1/s) | KD (M) |
|---|---|---|---|
| VHH1-Fc | 2.93E+04 | 7.33E-03 | 2.5E-07 |
| VHH2-Fc | 3.02E+04 | 2.32E-03 | 7.71E-08 |
| VHH6-Fc | 5.57E+04 | 8.54E-03 | 1.53E-07 |
| VHH8-Fc | 4.86E+04 | 7.35E-03 | 1.51E-07 |
| VHH13-Fc | 5.05E+04 | 4.87E-03 | 9.64E-08 |
| Utomilumab | 6.84E+05 | 6.89E-03 | 1.01E-08 |
| Urelumab | 4.96E+05 | 1.59E-03 | 3.22E-09 |

### Example 4: FACS Analysis of Binding of 4-1BB-VHH-Fc Fusion Proteins to 4-1BB-Expressing Cells

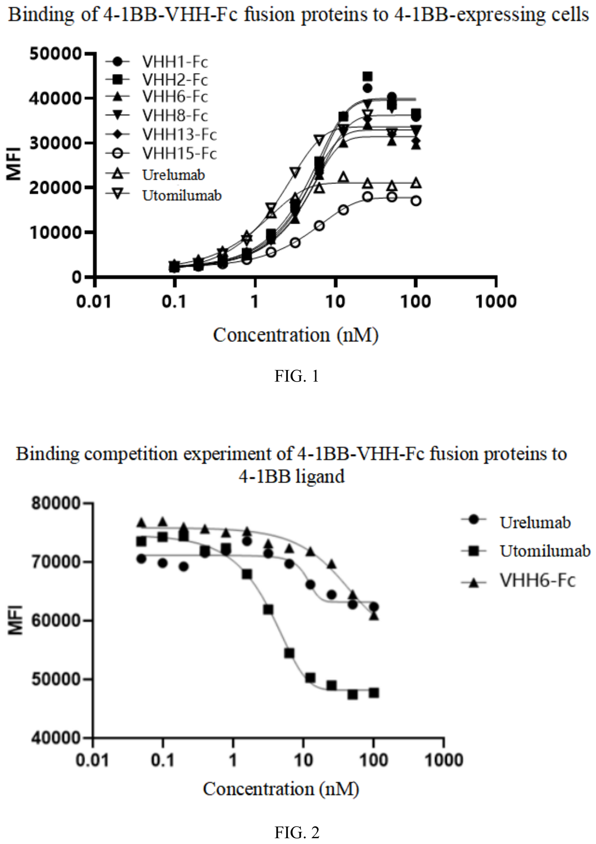

The binding of the 4-1BB-VHH-Fc fusion proteins to 4-1BB-expressing HEK-293T (293T-4-1BB) cells was assessed using an assay based on fluorescence-activated cell sorting (FACS). Different fusion protein samples (2-fold serial dilution starting from 100 nM, a total of 11 concentrations) were prepared as primary antibodies for FACS analysis. 293T-4-1BB cells were dissociated from the adherent culture flask and mixed with the fusion protein samples with different concentrations (all in a 96-well plate). Urelumab and utomilumab were used as anti-4-1BB antibody positive controls. The mixtures were equilibrated at 4 °C for 60 min and washed three times with PBS buffer. A phycoerythrin (PE)-conjugated goat-anti-human IgG Fc antibody (Invitrogen) was then added as a secondary antibody, and the mixtures were equilibrated at 4 °C in a dark place for 30 min. The cells were washed again with PBS buffer and analyzed by flow cytometry. Data were analyzed using non-linear regression and GraphPad PRISM 8 (GraphPad Software, San Diego, CA). The results are shown in FIG. 1. The FACS binding assay shows that the fusion proteins were all able to bind to 4-1BB-expressing cells.

Preparation method for HEK-293T (293T-4-1BB) cells: HEK-293T cells were transfected with a luciferase reporter gene vector containing an NF-κB promoter. The cells were subjected to resistance screening with the addition of hygromycin (100 µg/mL) to obtain a stable cell line. Cells of the cell line were transfected with a vector expressing h4-1BB and subjected to screening with the addition of puromycin (0.3 µg/mL) to obtain a 4-1BB/NF-κB reporter gene-HEK-293T stable cell line. The amino acid sequence of h4-1BB is set forth in SEQ ID NO: 28.

### Example 5: FACS Analysis of Inhibition of Binding of 4-1BB to Its Ligand 4-1BBL by 4-1BB-VHH-Fc Fusion Proteins

To assess the ability of the fusion proteins to inhibit the binding of 4-1BB to its ligand 4-1BBL (competition test), 4-1BB-VHH-Fc fusion protein samples were prepared (2-fold serial dilution starting from 100 nM, a total of 12 concentrations). Human 4-1BB-expressing 293T cells (i.e., HEK-293T (293T-4-1BB), see Example 4 for the preparation method) were dissociated from the adherent culture flask and mixed with each of the fusion protein samples with different concentrations and 1 nM biotinylated h4-1BBL protein (Sino Biological, Cat. No. 15693-H42H-B). Utomilumab and urelumab, which are functionally similar, were used as anti-4-1BB antibody positive controls. The mixtures were equilibrated at 4 °C for 60 min and washed three times with PBS buffer. A PE-streptavidin secondary antibody (Invitrogen) was then added to the mixtures, and the mixtures were equilibrated at 4 °C in a dark place for 30 min. Subsequently, the cells were washed with PBS buffer and analyzed by flow cytometry.

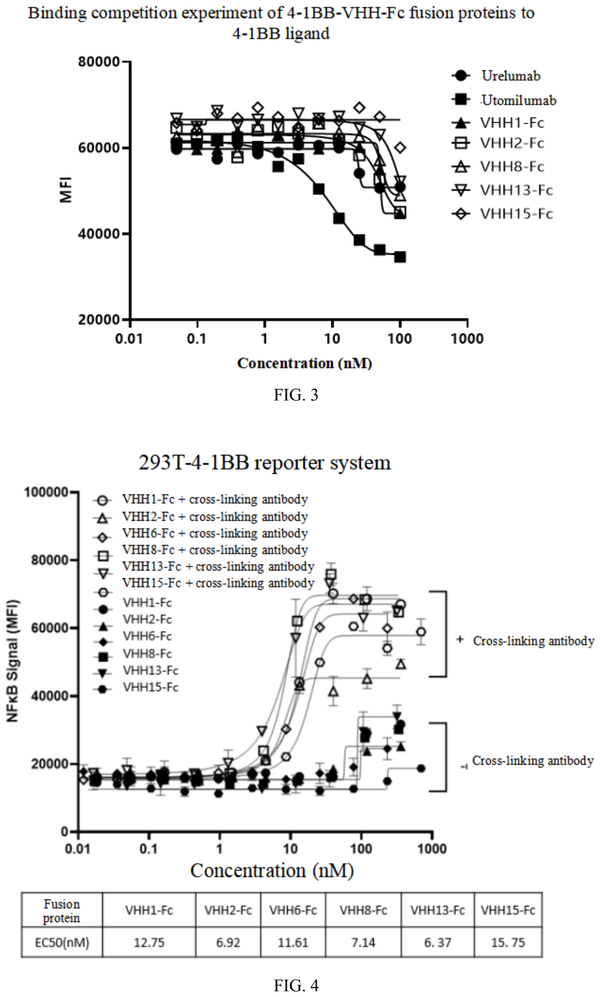

Data were analyzed using non-linear regression and GraphPad PRISM 8 (GraphPad Software, San Diego, CA). The competition assay shows that utomilumab demonstrated the ability to effectively inhibit the 4-1BB/4-1BBL interaction at low concentrations (0.1-10 nM/mL), while the different fusion protein samples and urelumab were unable to inhibit the 4-1BB/4-1BBL interaction even at high concentrations (10-100 nM/mL). The binding data in FIG. 2 and FIG. 3 indicate that neither the 4-1BB-VHH-Fc fusion proteins nor urelumab blocked the binding of 4-1BB to 4-1BBL.

### Example 6: In Vitro Functional Assay of 4-1BB-VHH-Fc Fusion Proteins

The activation of the 4-1BB pathway by 4-1BB-VHH-Fc samples can be studied using a variety of bioassays that monitor T cell proliferation, IFN-γ release, IL-2 secretion, or reporter gene expression driven by signaling in the 4-1BB pathway.

For an *in vitro* bioactivity assessment, 6 different 4-1BB-VHH-Fc fusion proteins were selected. 293T-4-1BB cells were used. The characterization of the bioactivity of anti-4-1BB agonistic antibodies in an assay based on 4-1BB cells is shown in FIG. 4. The specific experiment was as follows: The different fusion proteins were separately mixed with an anti-human IgG (Fc-specific) (goat antibody, Sigma-Aldrich, Cat. No. I2136) at a ratio of 1:1, or the anti-human IgG (Fc-specific) was not added. 293T-4-1BB cells were well mixed with serially diluted fusion protein samples (3-fold serial dilution starting from 500 nM, having 10 concentrations), and the mixtures were incubated in a cell incubator. After about 6 h of stimulation, a Bio-Lite^{™} Luciferase Assay System luciferase reagent (Vazyme) was added to the cells to measure NF-κB activity. Data were analyzed using non-linear regression and GraphPad PRISM 8 (GraphPad Software, San Diego, CA), and EC₅₀ values were calculated. The anti-human IgG (Fc-specific) (goat antibody, polyclonal antibody) was able to specifically bind to the Fc regions of at least two 4-1BB-VHH-Fc fusion proteins to produce an aggregation effect on the fusion proteins, thereby activating the 4-1BB activation activity of the fusion proteins. As shown in FIG. 4, the reporter gene assay shows that the fusion proteins were all able to effectively activate NF-κB signaling in the presence of the anti-human IgG (Fc-specific), but did not activate NF-κB signaling when they were alone; by way of contrast, the control antibody urelumab alone was able to effectively activate NF-κB signaling, indicating that the fusion proteins of the present disclosure require cross-linking for activation and thus have higher safety.

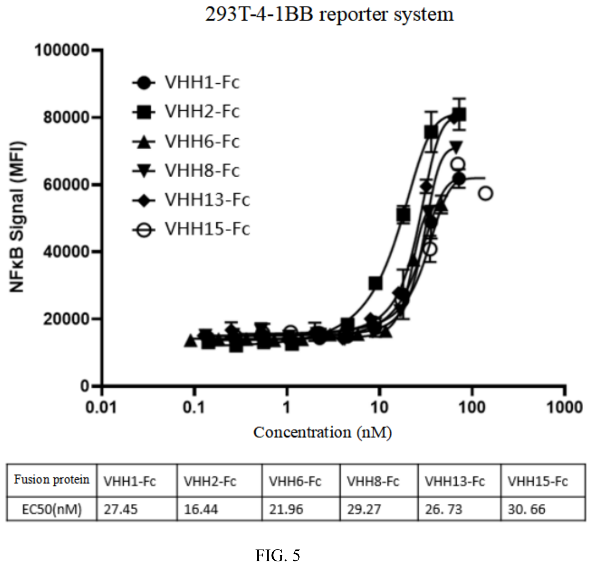

Another case can also reflect that the different fusion proteins can effectively activate NF-κB signaling in the presence of cross-linking. In this case, fusion protein samples (2-fold serial dilution starting from 100 nM, having 10 concentrations) were prepared and placed in an ELISA plate at 4 °C overnight for coating, so that the fusion proteins were cross-linked to the ELISA plate. Then 293T-4-1BB cells were added. After about 6 h of stimulation, a Bio-Lite^{™} Luciferase Assay System luciferase reagent (Vazyme) was added to the cells to measure NF-κB activity. Data were analyzed using non-linear regression and GraphPad PRISM 8 (GraphPad Software, San Diego, CA), and EC₅₀ values were calculated. The reporter gene assay in FIG. 5 also shows that the fusion proteins were all able to effectively activate NF-κB signaling after cross-linking.

## Claims

1. A 4-1BB-binding protein, comprising a heavy chain variable region, wherein the heavy chain variable region comprises one or more of the following complementarity determining regions (CDRs): an HCDR1 set forth in SEQ ID NO: 7, an HCDR2 set forth in any one of SEQ ID NOs: 8-13 or SEQ ID NOs: 31-36, and an HCDR3 set forth in any one of SEQ ID NOs: 14-19 or SEQ ID NOs: 37-42.

2. The 4-1BB-binding protein according to claim 1,
wherein the heavy chain variable region comprises an HCDR1 set forth in SEQ ID NO: 7, an HCDR2 set forth in SEQ ID NOs: 8-13, and an HCDR3 set forth in any one of SEQ ID NOs: 14-19; or
the heavy chain variable region comprises an HCDR1 set forth in SEQ ID NO: 7, an HCDR2 set forth in SEQ ID NO: 8, and an HCDR3 set forth in any one of SEQ ID NOs: 14-17; or
the heavy chain variable region comprises an HCDR1 set forth in SEQ ID NO: 7, an HCDR2 set forth in SEQ ID NO: 9, and an HCDR3 set forth in SEQ ID NO: 14 or 19; or
the heavy chain variable region comprises an HCDR1 set forth in SEQ ID NO: 7, an HCDR2 set forth in SEQ ID NO: 10, and an HCDR3 set forth in SEQ ID NO: 14 or 16; or
the heavy chain variable region comprises an HCDR1 set forth in SEQ ID NO: 7, an HCDR2 set forth in SEQ ID NO: 11 or 12, and an HCDR3 set forth in SEQ ID NO: 16; or
the heavy chain variable region comprises an HCDR1 set forth in SEQ ID NO: 7, an HCDR2 set forth in SEQ ID NO: 13, and an HCDR3 set forth in SEQ ID NO: 18 or 19; or
wherein the heavy chain variable region comprises an HCDR1 set forth in SEQ ID NO: 7, an HCDR2 set forth in SEQ ID NOs: 31-36, and an HCDR3 set forth in any one of SEQ ID NOs: 37-42; or
the heavy chain variable region comprises an HCDR1 set forth in SEQ ID NO: 7, an HCDR2 set forth in SEQ ID NO: 31, and an HCDR3 set forth in any one of SEQ ID NOs: 37-40; or
the heavy chain variable region comprises an HCDR1 set forth in SEQ ID NO: 7, an HCDR2 set forth in SEQ ID NO: 32, and an HCDR3 set forth in SEQ ID NO: 37 or 42; or
the heavy chain variable region comprises an HCDR1 set forth in SEQ ID NO: 7, an HCDR2 set forth in SEQ ID NO: 33, and an HCDR3 set forth in SEQ ID NO: 37 or 39; or
the heavy chain variable region comprises an HCDR1 set forth in SEQ ID NO: 7, an HCDR2 set forth in SEQ ID NO: 34 or 35, and an HCDR3 set forth in SEQ ID NO: 39; or
the heavy chain variable region comprises an HCDR1 set forth in SEQ ID NO: 7, an HCDR2 set forth in SEQ ID NO: 36, and an HCDR3 set forth in SEQ ID NO: 41 or 42.

3. The 4-1BB-binding protein according to claim 1 or 2, wherein the heavy chain variable region comprises an amino acid sequence set forth in any one of SEQ ID NOs: 1-6 and 29-30, or an amino acid sequence having at least 80% identity compared to the amino acid sequence set forth in any one of SEQ ID NOs: 1-6 and 29-30, or an amino acid sequence having one or more conservative amino acid substitutions compared to the amino acid sequence set forth in any one of SEQ ID NOs: 1-6 and 29-30.

4. A 4-1BB-binding protein, comprising an amino acid sequence set forth in any one of SEQ ID NOs: 1-6 and 29-30.

5. The 4-1BB-binding protein according to any one of claims 1-4, wherein the 4-1BB-binding protein is a single-domain antibody, a heavy-chain antibody, a bispecific antibody, a multispecific antibody, a Fab, a Fab', a F(ab')2, an Fv, or an scFv.

6. The 4-1BB-binding protein according to any one of claims 1-4, wherein the 4-1BB-binding protein is a fusion protein and further comprises an Fc region; preferably, the Fc is an Fc of an immunoglobulin, such as an IgG1 Fc; or the Fc is a variant Fc having one or more amino acid modifications, such as substitutions, deletions, or insertions.

7. The binding protein according to claim 6, wherein in the fusion protein, the C-terminus of the heavy chain variable region and the N-terminus of the Fc region are further linked by a peptide linker.

8. The binding protein according to claim 7, wherein a sequence of the peptide linker is (GₘS)ₙ, wherein each m is independently 2, 3, 4, or 5, and n is independently 1, 2, 3, 4, 5, or 6.

9. A 4-1BB-binding protein, comprising an amino acid sequence set forth in any one of SEQ ID NOs: 22-27.

10. A biomaterial, wherein the biomaterial is
1) a nucleic acid molecule encoding the 4-1BB-binding protein according to any one of claims 1-9;
2) an expression vector comprising the nucleic acid molecule encoding the 4-1BB-binding protein according to any one of claims 1-9; or
3) a host cell comprising the nucleic acid molecule or the expression vector encoding the 4-1BB-binding protein according to any one of claims 1-9.

11. A pharmaceutical composition, comprising the 4-1BB-binding protein according to any one of claims 1-9 and a pharmaceutically acceptable carrier.

12. Use of the 4-1BB-binding protein according to any one of claims 1-9, the biomaterial according to claim 10, or the pharmaceutical composition according to claim 11 in the preparation of a medicament for treating or preventing a disease, wherein preferably, the disease is a tumor, an autoimmune disease, or an inflammatory disease.
